Europäisches Patentamt

European Patent Office

Office européen des brevets

(11) Publication number: **0 161 681**
A2

# EUROPEAN PATENT APPLICATION

(21) Application number: 85105991.5

(22) Date of filing: 15.05.85

(51) Int. Cl.⁴: **C 08 L 5/12,** A 61 K 9/70
// (C08L5/12, 5:14)

(30) Priority: 17.05.84 JP 99419/84
18.05.84 JP 99816/84
21.05.84 JP 101929/84

(43) Date of publication of application: 21.11.85
Bulletin 85/47

(84) Designated Contracting States: **CH DE FR GB LI NL**

(71) Applicant: **MITSUBISHI ACETATE CO., LTD.,**
**3-19 Kyobashi 2-chome, Chuo-ku Tokyo 104 (JP)**

(72) Inventor: **Mochida, Naoki, B-402, Kaigandori 3, Toyama**
**City Toyama (JP)**
Inventor: **Tabuchi, Takashi, B-204, Kaigandori 3, Toyama**
**City Toyama (JP)**
Inventor: **Sawada, Hiroaki, 744 Uchudani, Kurobe City**
**Toyama (JP)**
Inventor: **Yamamoto, Osamu, 36-5-306, Kyodo 5-chome**
**Setagaya-ku, Tokyo (JP)**

(74) Representative: **Hoffmann, Klaus, Dr. rer. nat. et al,**
**Hoffmann . Eitle & Partner Patentanwälte**
**Arabellastrasse 4, D-8000 München 81 (DE)**

(54) Gel plate and process for preparing same.

(57) A gel plate contains a polysaccharide and an aqueous
solution of polyhydric alcohol. An intermediate gel plate
product is prepared from an aqueous solution of polyhydric
alcohol containing not more than 50% by weight of a polysaccharide dissolved therein, a ratio of water to polyhydric
alcohol in the aqueous solution bein 95:5 to 40:60. The inter-
mediate gel plate product is processed to lower the ratio of
water to polyhydric alcohol to a predetermined level to form
the gel plate.

## GEL PLATE AND PROCESS FOR PREPARING SAME

This invention relates to a gel plate containing a polysaccharide as a main component and a process for preparing the same.

Various polysaccharide gels such as agar-agar are known in the art. None of them are suited for industrial use since they do not have a sufficient strength, and they are used mainly for food. For example, in the case of a poultice to which a gel plate under consideration is applicable, a base material for a poultice component, a percutaneous absorbent and so on comprises a gel made of polyacrylic acid and gelatin, and minerals such as kaolin. However, such a gel-based base material is thermoplastic and has a low gel-melting temperature. Therefore, such a base material is susceptible to a run in use, and in addition, when it is separated from the skin of the patient, part of the base material remains there as a result of the run. Further, the base material has such a low gel strength that it must be supported by a suitable support such as a film and a woven or a non-woven cloth. Further, such a gel has a high viscosity, so that the body hair is pulled when separating the base material from the skin, thereby causing the patient much pain.

With the above deficiencies in view, it is an object of this invention to provide a gel plate which has a sufficient strength that it can be used as a base material of a poultice and obviates the need for a support for the gel.

According to a first aspect of the present invention, there is provided a polysaccharide gel plate which comprises a polysaccharide and an aqueous solution of polyhydric alcohol.

Any polysaccharide can be used so long as it has a gel-formability. Examples of polysaccharides include carrageenan, Furcellaran and agar-agar. Also, even those polysaccharides which have no gel-formability by itself can be used so long as they exhibit a gel-formability in combination with other substances. These polysaccharides having a gel-formability alone or in combination with other substance are hereinafter referred to as "polysaccharide having a gel-formability". Examples of the combination of polysaccharide and other substance include a combination of guar gum and a boron compound, a combination of pectin and alkaline earth metal, a combination of pectin and sugar, a combination of tamarind gum and sugar, a combination of tamarind gum and alcohol, and a combination of konjac mannan and alkaline earth metal.

The polysaccharide having a gel-formability may be a polysaccharide derivative such as a hydroxypropyl derivative of guar gum.

Among the polysaccharides, carrageenan and its mixture with other polysaccharide are preferred because of its high stretchability. It is preferred that the carrageenan content of such a mixture should be at least 20 %.

Preferably, the gel plate contains carrageenan and galactomannan. In this case, the ratio of carrageenan to galactomannan is preferably 20 : 80 to 99 : 1 in order to enhance stretchability and processability of the polysaccharides. With the increase of carrageenan, the rupture strength, stretchability and transparency of the gel plate are also increased. However, with an undue increase of carrageenan, these performances begin to be lowered after respective peaks. Therefore, it is preferred that the ratio of carrageenan to galactomannan should not exceed 20/80. More preferably, this ratio is in the range of between 35 : 65 and 95 : 5. Any type of galactomannan can be used, and locust bean gum is most preferred.

Examples of polyhydric alcohol include sorbitol, glucose, sucrose, ethyleneglycol, diethyleneglycol, triethyleneglycol, polyethyleneglycol, propyleneglycol, polypropyleneglycol, butanediol and glycerol. The polyhydric alcohol is effective for restraining the formation of a film on a surface of the gel plate due to the drying thereof so as to enhance a sustained release of the medical component in the gel plate.

The alcohol concentration of the aqueous solution and the aqueous solution content in the gel plate are not strictly specified. Also, the water may be volatilized from the gel plate by drying after it is prepared, and therefore it is rather difficult to specify the alcohol concentration of the aqueous solution and the aqueous solution content in the gel plate. However, in the preparation of the gel plate, it is preferred from the viewpoint of operability that a ratio of water to alcohol is in the range of between 95 : 5 and 40 :

60. If this ratio is less than the above-mentioned lower limit, that is to say, the water content becomes less than the above lower limit, it is difficult to dissolve the polysaccharide substantially uniformly. On the other hand, if the water content becomes greater than the above upper limit, the water retention of the gel plate tends to be lowered.

The concentration of the polysaccharide in the aqueous solution of polyhydric alcohol is preferably 0.1 to 50 % by weight and more preferably 0.1 to 30 % by weight. If this concentration is above 50 %, it is difficult to obtain a uniform solution. And, if this concentration is below 0.1 %, the gel-formability of the polysaccharide can not be achieved to a satisfactory level.

The gel plate can contain a medical component, a watersoluble polymer, an inorganic salt and so on. Any medical component can be used so long as it exhibits a percutaneous absorbency. Examples of such medical components include skin stimulants such as methyl salicylate, ethyleneglycol salicylate, camphor, menthol and capsicum, an antiphlogistic analgesic such as prednisolone and indomethacin, and antibiotics.

The gel plate according to the present invention can be prepared by dispersing a polysaccharide having a gel-formability such as carrageenan in an aqueous solution of polyhydric alcohol to form a dispersion, heating the dispersion to dissolve the polysaccharide to form a solution, forming the resultant hot solution into a flat plate by extrusion using a slit or flow casting, and cooling the plate.

In the case where a polysaccharide having no gel-

- 5 -    **0 161 681**

formability by itself is used in combination with the coacting substance, preferably, an aqueous polyhydric alcohol solution of the polysaccharide is first prepared, and the coacting substance is added to the solution to form a mixture solution which is formed into a flat plate.

The medical component may be added to the hot solution. Alternatively, the gel plate so prepared may be coated with or impregnated with a solution or a dispersion of a medical component. In the latter case, it is preferred that prior to applying the medical component to the gel plate, part of the the water is volatilized from the gel plate by heating so that the medical component can be sufficiently absorbed in the gel plate.

Therefore, according to a second aspect of the present invention, there is provided a process for preparing a gel plate containing an aqueous solution of polyhydric alcohol which comprises the steps of preparing an intermediate gel plate product from an aqueous solution of polyhydric alcohol containing not more than 50 % by weight of a polysaccharide dissolved therein, a ratio of water to polyhydric alcohol in the aqueous solution being 95 : 5 to 40 : 60; and processing the intermediate gel plate product to lower the ratio of water to polyhydric alcohol to a predetermined level to form the gel plate. The gel plate so prepared has a high absorbency, so that the medical component can be sufficiently retained in the gel plate even at low temperatures.

In the present process, a ratio of polysaccharide to polyhydric alcohol is preferably 1 : 500 to 20 :1, and more preferably 1 : 50 to 10 : 1. If the polyhydric alcohol exceeds the upper limit, it is difficult to dissolve the polysaccharide uniformly in the aqueous solution of the polyhydric alcohol. On the other hand, if the polyhydric alcohol is below the lower limit, the water retention of the gel plate is lowered, and when the gel plate is dried, it tends to become brittle.

The gel plate contains at least 50 % by weight of the aqueous polyhydric alcohol solution in which the ratio of water to polyhydric alcohol is 95 : 5 to 40 : 60. The lowering of this ratio can be effected by heating and drying the gel plate. Usually, it is considered that it is not desirable to heat and dry a polysaccharide of the type having a gel-formability by itself because it forms a reversible gel. However, the gel plate according to the present invention is not subjected to deformation even when it is heated at elevated temperatures of 60 to 120$^{\circ}$C. When the gel plate is heated to be dried, it is desirable that the polyhydric alcohol is not volatilized. Therefore, preferably, the gel plate is dry heated at temperatures of 50 to 130$^{\circ}$C. A hot plate or the like can be used for this purpose. Also, vacuum drying can be employed.

When the water content of the gel plate is lowered, the ability of the gel plate to absorb an aqueous solution of a medical component or the like is enhanced. The ratio of water to polyhydric alcohol in the gel plate as a final product is preferably not more than 40 : 60.

The invention will now be illustrated by way of the following Examples. In the Examples, transmittance is expressed in percentage, using light having a wavelength of 660 nm and air as a control. Transparency is expressed in transmittance (%) at a thickness of 10 mm according to the Lambert-Beer's law. Thickness is measured by Peacock thickness gauge. Strength and stretchability are determined according to tensile tests at a test piece width of 10 mm, the inter-plunger length of 20 mm and a pulling speed of 30 cm/min, using a rheometer. The strength is expressed in $kg/cm^2$ by dividing the breaking stress by the initial cross-sectional area, and the stretchability is expressed in percentage by dividing the breaking elongation by the initial inter-plunger length. Bonding strength is expressed by a peeling stress determined through a rheometer by fixing a square test piece of 2.5 x 2.5 $cm^2$ with respect to a bottom surface of a plunger in parallel relation thereto, bonding the bottom surface having a diameter of 15 mm to the test piece, and moving the bottom surface off the test piece.

### Example 1

30g of purified carrageenan whose counter-cation was potassium was dispersed in 700 ml of water, and the dispersion was heated at 85°C with stirring to form a uniform solution. Then, 300 ml of glycerin was added to the solution, and this mixture was stirred while being heated to form a uniform solution. Then, the hot solution was flow cast into a gel plate having a thickness of 660 μm using a thin layer chromatograph applicator. The gel plate so obtained had a transmittance of 43 % and a transparency of 27.5 %. Thus, the gel plate exhibited a very good transparency. The gel plate also had a strength of 1.62 $kg/cm^2$, a stretchability of 110.5

%, and a bonding strength of 11 g/cm$^2$. When the gel plate was stuck to a wrist joint part, it offered a very good adhesion and a refreshing feeling. The gel plate exhibited a very good response to the stretching of the wrist joint. Thus, it was realized that the gel plate could be satisfactorily used as a base material for poultice without the need for any support such as a film and a cloth. In addition, the gel plate was inconspicuous when applied to the wrist because of its good transparency.

Example 2

A gel plate was prepared according to the procedure of Example 1. The gel plate was dried on a hot plate at 120$^O$C for 10 minutes, so that it was reduced in thickness into 270 μm. The resultant gel plate was subjected to heating loss at 105$^O$C for 5 hours from which it was determined that the gel plate contained 7 % by weight of carrageenan, 73 % by weight of glycerin and 20 % by weight of water. The gel plate had a transparency of 15 % and therefore exhibited a very good transparency. The gel plate had a strength of 14 kg/cm$^2$, a stretchability of 145 % and a bonding strength of 21 kg/cm$^2$. When the gel plate was dipped in water at 25$^O$C for 10 minutes, the gel plate absorbed 2.9 times water per weight of the gel plate. When the gel plate was stuck to a wrist joint part, it offered a very good adhesion and a refreshing feeling. The gel plate exhibited a very good response to the stretching of the wrist joint. Thus, it was realized that the gel plate could be satisfactorily used as a base material for poultice without the need for any support such as a film and a cloth.

In addition, the gel plate was inconspicuous when applied to the wrist because of its good transparency.

Example 3

The gel plate was prepared according to the procedure of Example 1 except that the counter-cation of the carrageenan was sodium. The gel plate had a transparency of 19 % and therefore exhibited a very good transparency. The gel plate had a strength of 0.89 kg/cm$^2$, a stretchability of 86.3 % and a bonding strength of 23 g/cm. When the gel plate was stuck to a wrist joint part, it offered a very good adhesion and a refreshing feeling. The gel plate exhibited a very good response to the stretching of the wrist joint. Thus, it was realized that the gel plate could be satisfactorily used as a base material for poultice without the need for any support such as a film and a cloth. In addition, the gel plate was inconspicuous when applied to the wrist because of its good transparency.

Example 4

A gel plate was prepared according to the procedure of Example 3. The gel plate was dried on a hot plate at 120$^o$C for 10 minutes, so that it was reduced in thickness into 300 μm. The resultant gel plate was subjected to heating loss at 105$^o$C for 5 hours from which it was determined that the gel plate contained 7% by weight of carrageenan, 73% by weight of glycerin and 20 % by weight of water. The gel plate had a transparency of 15 %, a strength of 14 kg/cm$^2$, a stretchability of 145 % and a bonding strength of 35 g/cm$^2$.

When the gel plate was stuck to a wrist joint part, it offered a very good adhesion and a refreshing feeling. The gel plate exhibited a very good response to the stretching of the wrist joint. Thus, it was realized that the gel plate could be satisfactorily used as a base material for poultice without the need for any support such as a film and a cloth. In addition, the gel plate was inconspicuous when applied to the wrist because of its good transparency.

As described above, the gel plate according to the present invention has a high strength and a high stretchability, and can be well suited for a base material for poultice since it has a good ability to absorb the medical component of the poultice. Particularly, in the case where the gel plate is used as a base material for poultice, the gel plate prepared by drying the intermediate product to lower the ratio of water to polyhydric alcohol exhibits a higher absorbency to the medical component than that which is not subjected to such drying and has the same water/polyhydric alcohol ratio. In addition, the gel plate has a good adhesion to the skin and is sufficiently elastic so that it can be well responsive to the stretching of the skin. Further, the gel plate will not cause a pain to the patient when separating the gel plate from the skin. And, as mentioned above, the gel plate has a sufficiently high strength that it can be used as the poultice base material without the need for any support. Further, the gel plate has such a good water retention that an effect imparted by the medical component of the poultice on the patient can last long.

0 161 681

CLAIMS:

1.　A gel plate comprising a polysaccharide and an aqueous solution of polyhydric alcohol.

2.　A gel plate according to claim 1, in which said polysaccharide contains carrageenan.

3.　A gel plate according to claim 1, in which said polysaccharide contains carrageenan and galactomannan.

4.　A gel plate according to claim 3, in which a ratio of carrageenan to galactomannan is 20 : 80 to 99 : 1.

5.　A gel plate according to claim 3 or claim 4, in which said galactomannan is locust bean gum.

6.　A process for preparing a gel plate containing an aqueous solution of polyhydric alcohol which comprises the steps of preparing an intermediate gel plate product from an aqueous solution of polyhydric alcohol containing not more than 50 % by weight of a polysaccharide dissolved therein, a ratio of water to polyhydric alcohol in the aqueous solution being 95 : 5 to 40 : 60; and processing the intermediate gel plate product to lower the ratio of water to polyhydric alcohol to a predetermined level to form the gel plate.

7.　A process according to claim 6, in which said polysaccharide polysaccharide contains carrageenan.

8.  A process according to claim 6, in which said the ratio of water to polyhydric alcohol in said gel plate is less than 40 : 60.

9.  A process according to claim 6, in which said processing is to dry said intermediate gel plate product.

10.  A process according to claim 6, further comprises the step of applying a medical component to said gel plate.